# EUROPEAN PATENT APPLICATION

(11) **EP 2 843 187 A1**
(43) Date of publication of application: **04.03.2015**
(21) Application number: 12748145.5
(22) Date of filing: 25.05.2012
(51) Int. Cl.: E21B 47/047, G01V 9/02

(54) **APPARATUS FOR TRACKING LOCATION OF SALT WATER BOUNDARY OF UNDERGROUND WATER**

(30) Priority: 26.04.2012 KR 20120043732
(71) Applicant: Korea Institute Of Geoscience & Mineral Resources, Daejeon 305-350 (KR)
(72) Inventor: KIM, Yongchel, Daejeon 305-350 (KR); KO, Kyung-Seok, Daejeon 305-768 (KR); KIM, Jung Su, Seoul 138-050 (KR)
(74) Representative: EP&C
(86) International application number: PCT/KR2012/004175
(87) International publication number: WO 2013/162115

(57) **Abstract**

Disclosed herein is a device for tracking the position of a freshwater-saltwater interface of underground water in the coastal area. The device includes a buoyancy adjustment unit which comprises a pipe that has an internal space and is closed on a lower end thereof, a sealing cap which is removably coupled to an upper end of the buoyancy adjustment unit, a variable metal member which is attached to the sealing cap, a cordless measurement sensor which is provided under the lower end of the buoyancy adjustment unit, and a perforated pipe which is coupled to the lower end of the buoyancy adjustment unit and covers the cordless measurement sensor. Distilled water is supplied into the buoyancy adjustment unit, and a buoyancy of the buoyancy adjustment unit is adjusted depending on the amount of distilled water.

## Description

### Technical Field

The present invention relates to a device for tracking the position of the freshwater-saltwater interface of underground water and an apparatus for installing the device.

### Background Art

Generally, in coastal areas, using an excessive amount of underground water reduces the level of underground water, causing salt water (sea water) to be drawn into the underground water and contaminate it so that the underground water may not be able to be used as water for agriculture or industrial use as well as drinking water.

Therefore, systems for monitoring underground water have been used, wherein an underground water monitoring well is formed in the coastal area, and a wire or wireless measuring sensor is installed at a specific fixed depth in the underground water monitoring well to measure the level, temperature, conductivity, etc. of underground water.

A freshwater-saltwater interface (an interface between underground water and salt water) varies depending on two kinds of phenomena. The first is a reduction in the thickness of a fresh water layer. The other is variation in the thickness of a salt water layer.

The thickness of the fresh water layer may be increased by rainfall or reduced (become thinner) by pumping in its vicinity.

The salt water layer varies in response to periodical variation in the sea level because of the tidal phenomenon.

Recently, the sea level is gradually increased due to global warming.

If, due to a variety of factors, the thickness of the fresh water layer becomes thinner than the reference level while the thickness of the salt water layer increases, the average level of the freshwater-saltwater interface moves up. If the thickness of the fresh water layer increases while the thickness of the salt water layer is reduced, the average level of the freshwater-saltwater interface is lowered.

As such, a variety of complex phenomena, e.g. pumping an excessive amount of underground water, rainfall, the tidal phenomenon and an increase in the sea level because of global warming, frequently vary the position of the freshwater-saltwater interface.

However, a conventional measuring sensor which is installed in the monitoring well in the coastal area is disposed at a fixed depth regardless of whether it is a wire or wireless type. It is difficult to measure a real time variation of the position of the freshwater-saltwater interface using the conventional method. Typically, when installing a device for tracking the position of a freshwater-saltwater interface of underground water into the monitoring well or removing it therefrom, an apparatus for installing the device thereinto or removing it therefrom is used. In the conventional technique, depending on the diameter of the monitoring well, it may be difficult to precisely dispose the apparatus just above the freshwater-saltwater interface position tracking device, thus making the installation or removal process difficult.

Moreover, to precisely dispose the freshwater-saltwater interface position tracking device at the interface between the salt water and the fresh water, the buoyancy of the device must be adjusted. For this, a tub which contains water having the same salt concentration as that of that salt water must be prepared, and the buoyancy of the device must be adjusted in the tub. However, it is not easy to adjust the salt concentration of water in the tub, and the work of adjusting the buoyancy of the device in the tub inconveniences a user.

### Disclosure

### Technical Problem

Accordingly, the present invention has been made keeping in mind the above problems occurring in the prior art, and an object of the present invention is to provide a device for tracking the position of a freshwater-saltwater interface of underground water using a neutral buoyancy mechanism which is installed in an underground water monitoring well of the coastal area such that a cordless measurement sensor that measures the level, temperature, conductivity, etc. of the underground water is moved along with the freshwater-saltwater interface in the monitoring well, and in which the buoyancy of the device can be adjusted by adjusting the amount of air in the buoyancy adjustment unit so that the adjustment of the buoyancy of the device can be made precise, and in which graduations which indicate the volume are formed on the circumferential outer surface of the buoyancy adjustment unit, whereby the work of adjusting the buoyancy of the device to the neutral buoyancy with respect to the freshwater-saltwater interface can be conducted by a calculative method so that the work can be facilitated.

Another object of the present invention is to provide an apparatus for installing or removing the freshwater-saltwater interface position tracking device that uses the neutral buoyancy mechanism and is able to move in response to the position of the freshwater-saltwater interface so as to measure the level, temperature, conductivity, etc. of the underground water, and in which a metal member which has a size corresponding to the diameter of the monitoring well is removably attached to the buoyancy adjustment unit or the freshwater-saltwater interface position tracking device, so that when the freshwater-saltwater interface position tracking device is removed from the monitoring well, even if the position of the device is not precisely figured out regardless of the diameter of the monitoring well, the operation of removing the device from the monitoring well can be facilitated.

### Technical Solution

In order to accomplish the above objects, in an aspect, the present invention provides a device for tracking a position of a freshwater-saltwater interface of underground water in a coastal area in such a way that a cordless measurement sensor is inserted, to track a position of a freshwater-saltwater interface, into a underground water monitoring well that is formed to measure the underground water, the device including: a buoyancy adjustment unit comprising a pipe having an internal space, the buoyancy adjustment unit being closed on a lower end thereof; a sealing cap removably coupled to an upper end of the buoyancy adjustment unit; a variable metal member attached to the sealing cap; a cordless measurement sensor provided under the lower end of the buoyancy adjustment unit; and a perforated pipe coupled to the lower end of the buoyancy adjustment unit, the perforated pipe covering the cordless measurement sensor, wherein distilled water is supplied into the buoyancy adjustment unit, and a buoyancy of the buoyancy adjustment unit is adjusted depending on an amount of distilled water.

The variable metal member may be extendable in a circumferential direction depending on a diameter of the underground water monitoring well.

The buoyancy adjustment unit may be made of a transparent material, and graduations may be marked on an outer surface of the buoyancy adjustment unit, whereby the buoyancy of the buoyancy adjustment unit is adjusted by calculating the amount of distilled water supplied into the buoyancy adjustment unit depending on a weight of the device.

Furthermore, an air discharge hole may be formed in a connector connecting the perforated pipe to the buoyancy adjustment unit so that air pockets are able to be discharged out of the perforated pipe through the air discharge hole during the installation in the well.

In another aspect, the present invention provides a device for tracking a position of a freshwater-saltwater interface of underground water in a coastal area in such a way that a cordless measurement sensor is inserted, to track a position of a freshwater-saltwater interface, into a underground water monitoring well that is formed to measure the underground water, the device including: a buoyancy adjustment unit comprising a pipe having an internal space, the buoyancy adjustment unit being closed on a lower end thereof; a sealing cap removably coupled to an upper end of the buoyancy adjustment unit; a cordless measurement sensor provided under the lower end of the buoyancy adjustment unit; and a connection member provided under the lower end of the buoyancy adjustment unit, the connection member being connected to the cordless measurement sensor, wherein distilled water is supplied into the buoyancy adjustment unit, and a buoyancy of the buoyancy adjustment unit is adjusted depending on an amount of distilled water.

In a further aspect, the present invention provides an apparatus for installing or removing a device for tracking a position of a freshwater-saltwater interface of underground water, the apparatus including: a power cable; a rotary cylinder around which the power cable is wound, the rotary cylinder being provided so as to be rotatable; a fixed cylinder disposed at a center of the rotary cylinder, the fixed cylinder functioning as a rotary shaft of the rotary cylinder; an electromagnet connected to a first end of the power cable; a first connection bar connected to a second end of the power cable, the first connection bar fixed in the rotary cylinder; a connection ring having a circumferential outer surface in contact with the first connection bar, the connection ring provided in the fixed cylinder; a watertight sealing member covering the electromagnet; and a variable metal member that is removably attached to the electromagnet.

The connection ring may be connected to a second connection bar on a circumferential inner surface of the fixed cylinder, wherein the second connection bar may be electrically connected to a power supply connected to the fixed cylinder so that power is applied from the power supply to the second connection bar.

The second connection bar may be disposed in a hollow space of the fixed cylinder.

The first connection bar, the connection ring and the second connection bar may comprise conductors.

The first connection bar may include a covering part that is an insulator.

In addition, a spring may be provided in the covering part so that the contact between the connection ring and the first connection bar that is rotating around the connection ring is maintained by elastic force of the spring.

In yet another aspect, the present invention provides a method of tracking a position of a freshwater-saltwater interface of underground water using a freshwater-saltwater interface position tracking device including: a buoyancy adjustment unit comprising a pipe having an internal space, the buoyancy adjustment unit being closed on a lower end thereof; a sealing cap removably coupled to an upper end of the buoyancy adjustment unit; a variable metal member attached to the sealing cap; a cordless measurement sensor provided under the lower end of the buoyancy adjustment unit; and a perforated pipe coupled to the lower end of the buoyancy adjustment unit, the perforated pipe covering the cordless measurement sensor, the method including: supplying distilled water into the buoyancy adjustment unit and adjusting a buoyancy of the buoyancy adjustment unit so that the device is disposed at a freshwater-saltwater interface of the underground water; and tracking variation in a position of the freshwater-saltwater interface using the cordless measurement sensor.

In still another aspect, the present invention provides a method of installing a freshwater-saltwater interface position tracking device using an installation apparatus, the installation apparatus including: a power cable; a rotary cylinder around which the power cable is wound, the rotary cylinder being provided so as to be rotatable; a fixed cylinder disposed at a center of the rotary cylinder, the fixed cylinder functioning as a rotary shaft of the rotary cylinder; an electromagnet connected to a first end of the power cable; a variable metal member attached to a lower end of the electromagnet; a first connection bar connected to a second end of the power cable, the first connection bar fixed in the rotary cylinder; and a connection ring having a circumferential outer surface in contact with the first connection bar, the connection ring provided in the fixed cylinder, the method including: applying power to the connection ring of the installation apparatus so that a magnetic field is formed on the electromagnet by the applied power, thus magnetizing the electromagnet; attaching the freshwater-saltwater interface position tracking device to the magnetized electromagnet; varying a diameter of the variable metal member depending on a diameter of the underground water monitoring well; and inserting the freshwater-saltwater interface position tracking device into the underground water monitoring well.

### Advantageous Effects

In a device for tracking a position of a freshwater-saltwater interface of underground water using a neutral buoyancy mechanism according to the present invention, a buoyancy adjustment unit which uses fluid and adjusts the buoyancy is provided with a cordless measurement sensor so that the device moves in response to the varying positions of a freshwater-saltwater interface. Thus, the device can more precisely take real-time measurements of the level, temperature, conductivity, etc. of the underground water of the coastal area.

Furthermore, the present invention provides an installation apparatus for the freshwater-saltwater interface position tracking device that has the neutral buoyancy mechanism and is able to move in response to the position of the freshwater-saltwater interface so as to measure the level, temperature, conductivity, etc. of the underground water. The installation apparatus uses an electromagnet to install the freshwater-saltwater interface position tracking device in an underground water monitoring well or remove it therefrom.

### Description of Drawings

Fig. 1 is a schematic view illustrating a device for tracking the position of a freshwater-saltwater interface of underground water which is installed in a monitoring well that measures underground water, according to the present invention;
Fig. 2 is a schematic view showing the operation of the freshwater-saltwater interface position tracking device in the monitoring well;
Fig. 3 is a perspective view of the freshwater-saltwater interface position tracking device according to the present invention;
Fig. 4 is an exploded perspective view of the freshwater-saltwater interface position tracking device according to the present invention;
Fig. 5 is of schematic views of a variable metal member of the freshwater-saltwater interface position tracking device according to the present invention;
Fig. 6 is an exploded sectional view of the freshwater-saltwater interface position tracking device according to the present invention;
Fig. 7 is an enlarged view showing an air discharge hole of the freshwater-saltwater interface position tracking device according to the present invention;
Fig. 8 illustrates another embodiment of the freshwater-saltwater interface position tracking device according to the present invention;
Fig. 9 is a view illustrating a method of measuring variation of the freshwater-saltwater interface using the freshwater-saltwater interface position tracking device according to the present invention;
Fig. 10 is of schematic views showing the operation of installing the freshwater-saltwater interface position tracking device according to the present invention;
Fig. 11 is a perspective view of an apparatus (A) for installing the freshwater-saltwater interface position tracking device according to the present invention;
Fig. 12 is a side view of the installation apparatus (A) according to the present invention;
Fig. 13 is an enlarged view showing an electromagnet of the installation apparatus (A) according to the present invention;
Fig. 14 is a sectional perspective view illustrating the installation apparatus (A) according to the present invention;
Fig. 15 is an enlarged view of the installation apparatus (A) according to the present invention;
Fig. 16 is a flowchart of a method of tracking the position of the freshwater-saltwater interface according to the present invention; and
Fig. 17 is a flowchart of a method of installing the freshwater-saltwater interface position tracking device according to the present invention.

### Best Mode

Hereinafter, preferred embodiments of the present invention will be described in detail with reference to the attached drawings. Reference now should be made to the drawings, in which the same reference numerals are used throughout the different drawings to designate the same or similar components. If in the specification, detailed descriptions of well-known functions or configurations would unnecessarily obfuscate the gist of the present invention, the detailed descriptions will be omitted.

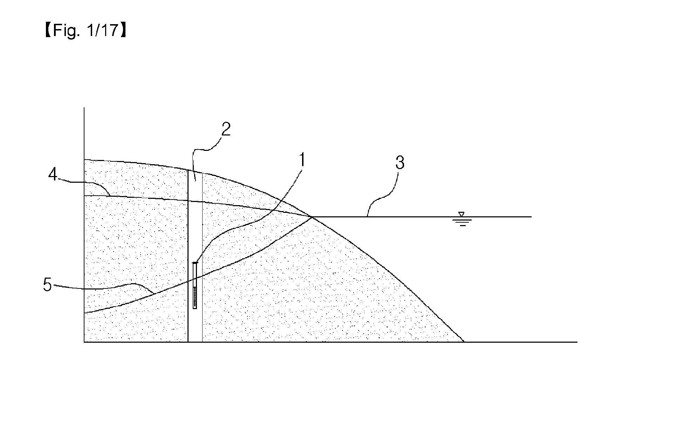

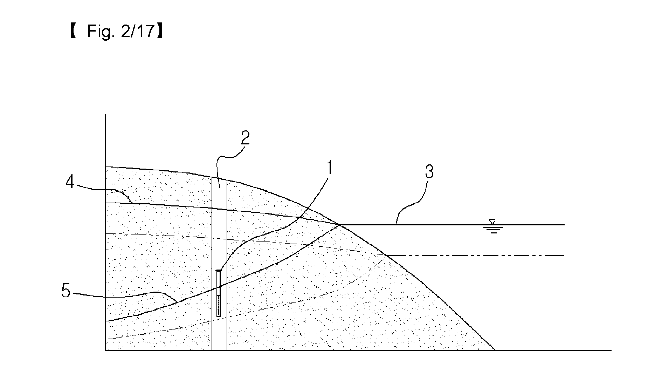

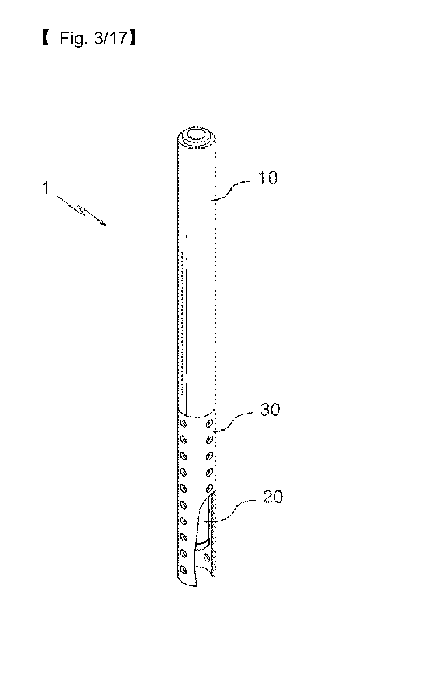

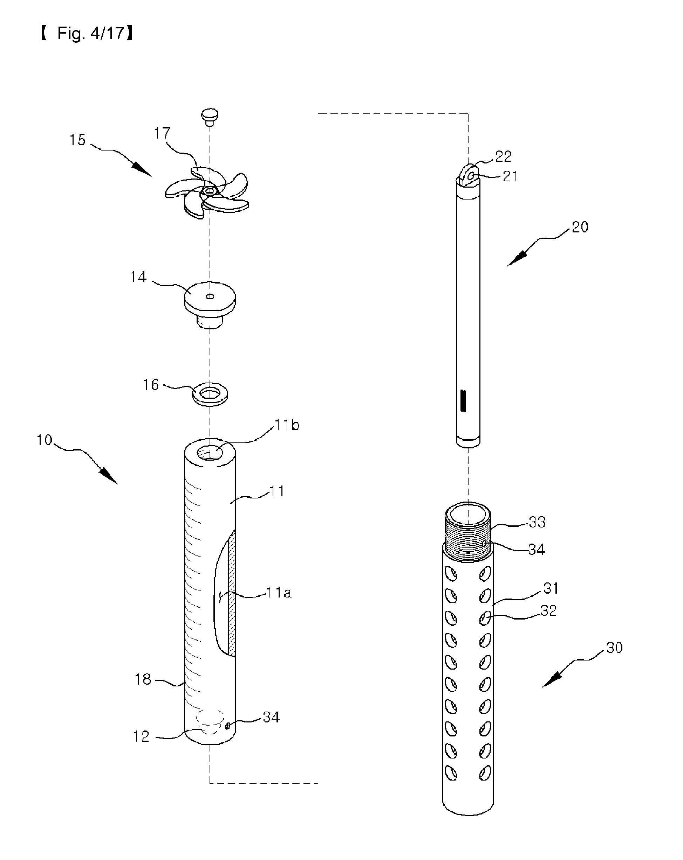

As shown in Figs. 1 and 2, a device 1 for tracking a position of a freshwater-saltwater interface of underground water according to the present invention is inserted into a monitoring well 2 which is formed in the coastal area to measure variations in the underground water. The freshwater-saltwater interface position tracking device 1 moves upwards or downwards depending on variation of a freshwater-saltwater interface. As shown in Figs. 3 and 4, the freshwater-saltwater interface position tracking device 1 includes a buoyancy adjustment unit 10 which adjusts the magnitude of buoyancy using fluid, a cordless measurement sensor 20 which is installed under a lower end of the buoyancy adjustment unit 10 to measure the water level, temperature, conductivity, etc. of underground water, and a perforated pipe 30 which is coupled to the lower end of the buoyancy adjustment unit 10 and covers the cordless measurement sensor 20.

As shown in Figs. 4 through 6, the buoyancy adjustment unit 10 includes a buoyancy body 11 which has an internal space 11a which can contain fluid therein. The buoyancy body 11 has the shape of a pipe (a tube) that is closed on a lower end thereof and has an opening 11b in the upper end thereof through which fluid can be supplied into the buoyancy body 11. A bracket 12 which has a through hole 12a formed to install the cordless measurement sensor 20 is provided in the lower end of the buoyancy body 11. An internal thread portion 13 is formed in the lower end of the buoyancy body 11 to install the perforated pipe 30 around the bracket 12. A sealing cap 14 which has a variable metal member 15 that is attracted to a magnet is installed on the upper end of the buoyancy body 11 so that after fluid is supplied into the internal space 11a, it can be sealed by the sealing cap 14.

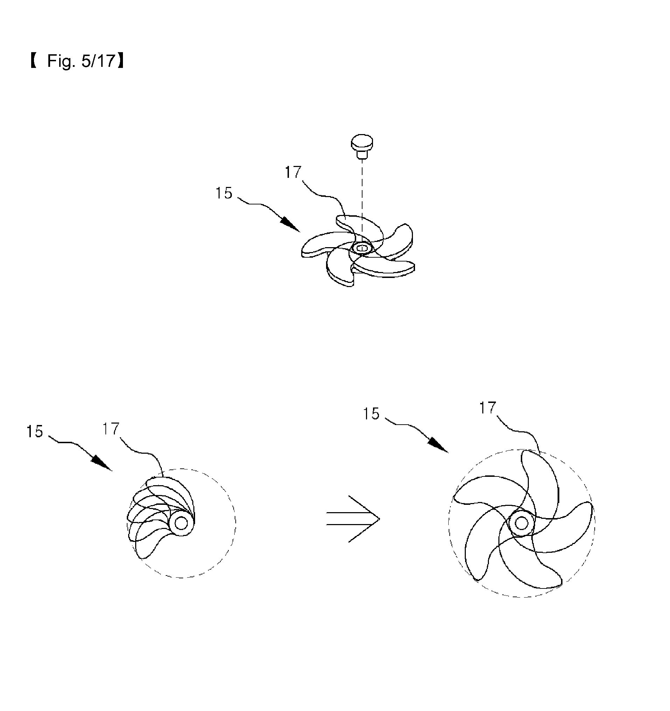

As shown in Fig. 5, the variable metal member 15 includes metal blades 17 which are provided so as to be unfoldable in a circumferential direction so that the size thereof can be expanded or contracted.

In the present invention, the variable metal member 15 is not limited to the above structure. For instance, the variation in the size may be embodied in such a way that the metal member 15 that is attached to the sealing cap 14 is replaced with another metal member of a different size, or then another metal member of a larger size is added to the metal member 15 that has been attached to the sealing cap 14.

The variable metal member 15 is provided on the sealing cap 14 so that it can be used when installing the freshwater-saltwater interface position tracking device 1 in the monitoring well 2 or removing the device 1 therefrom. That is, to install the device 1 in the monitoring well 2 or remove it therefrom, an installation apparatus A for the freshwater-saltwater interface position tracking device which will be explained later herein can use an electromagnet 100 to hold the device 1. Here, the electromagnet 100 of the installation apparatus A must be precisely disposed above the variable metal member 15 so that the electromagnet 100 can hold the freshwater-saltwater interface position tracking device 1 using magnetic force and remove it from the monitoring well.

If the diameter of the variable metal member 15 is smaller than that of the monitoring well 2, it is difficult for the electromagnet 100 of the installation apparatus A to be disposed precisely above the variable metal member 15, making the removal of the device 1 from the monitoring well 2 difficult. Therefore, the variable metal member 15 is preferably set such that the diameter thereof is slightly smaller than that of the monitoring well 2. Then, regardless of the location at which the electromagnet 100 of the installation apparatus A is positioned in the monitoring well 2, the operation of removing the device 1 from the monitoring well 2 can be facilitated.

The variable metal member 15 may be coupled to the sealing cap 14 by a screw. Alternatively, a protrusion provided with an external thread may be provided on the variable metal member 15 while an internal thread is formed in the upper end of the sealing cap 14 so that the variable metal member 15 is coupled to the sealing cap 14 by the engagement between the internal thread and the external thread.

An internal thread and an external thread that correspond to each other are respectively formed in the opening 11b and on the sealing cap 14. An airtight rubber packing 16 is interposed between the opening 11b and the sealing cap 14. Fluid 40 is supplied into the internal space 11a through the opening 11b to adjust the magnitude of the buoyancy before the internal space 11a is sealed by the sealing cap 14.

The cordless measurement sensor 20 is a typical sensor that measures the water level, temperature, conductivity, etc. of underground water in a coastal area. The cordless measurement sensor 20 measures variations in the underground water and sends wireless communication signals to a recording device that is installed on the ground, thus enabling an observer on the ground to monitor variations in the position of the freshwater-saltwater interface.

As shown in Fig. 4, a connector 21 that has a through hole 22 is provided on an upper end of the cordless measurement sensor 20 so that the cordless measurement sensor 20 can be coupled to the bracket 12 of the buoyancy adjustment unit 10 by the connector 21.

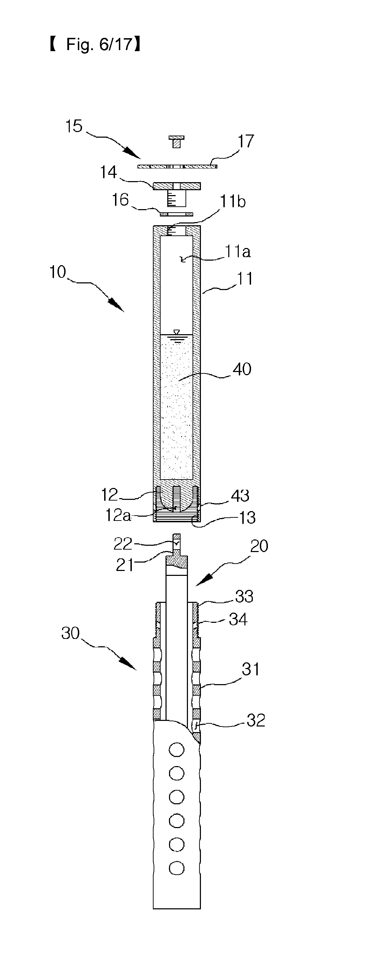

As shown in Fig. 6, the cordless measurement sensor 20 that has the above-mentioned construction is coupled to the lower end of the buoyancy adjustment unit 10 by a method in which the through hole 22 of the connector 21 is aligned with the through hole 12a of the bracket 12 of the buoyancy adjustment unit 10 and then a pin is inserted into the through holes 22 and 12.

The perforated pipe 30 encloses the cordless measurement sensor 20 and protects it. As shown in Fig. 4, the perforated pipe 30 allows underground water and salt water to access the cordless measurement sensor 20 that is disposed in the perforated pipe 30.

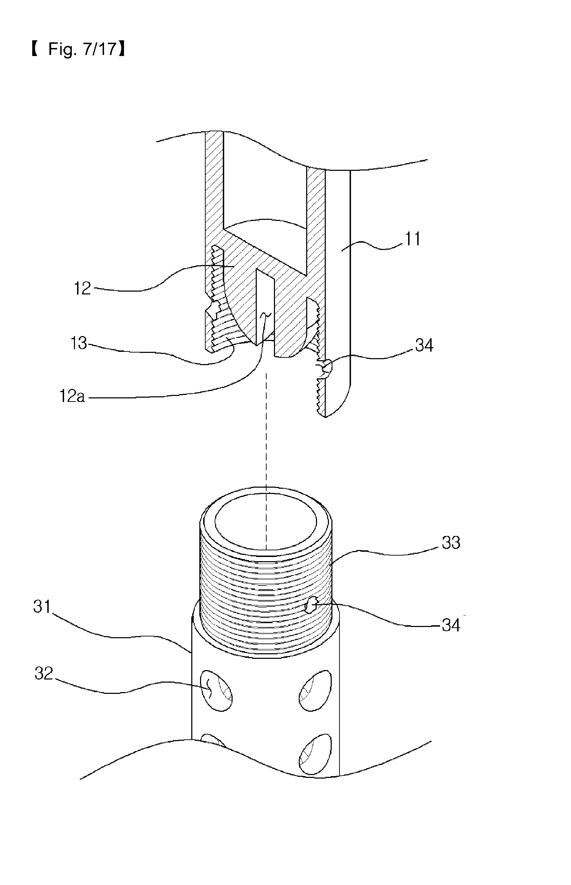

As shown in Figs. 6 and 7, a plurality of inlet holes 32 are formed in a circumferential surface of a perforated pipe body 31 so that underground water and salt water is drawn into the perforated pipe body 31 and is brought into contact with the cordless measurement sensor 20. An external threaded portion 33 is formed on an upper end of the perforated pipe body 31. Thereby, the perforated pipe body 31 can be coupled to the buoyancy body 11 by the engagement between the external threaded portion 33 and the internal thread portion 13 formed in the lower end of the buoyancy body 11.

The inlet holes 32 are arranged in the longitudinal direction of the perforated pipe body 31.

When the freshwater-saltwater interface position tracking device 1 is inserted into the monitoring well 2, underground water and salt water is drawn first into the inlet holes 32 that are disposed in the lower end of the perforated pipe body 31, while air that has been in the perforated pipe body 31 is discharged to the outside through the inlet holes 32 that are disposed in the upper end of the perforated pipe body 31.

However, a fine space is formed in a threaded coupling portion at which the external threaded portion 33 that is formed on the upper end of the perforated pipe body 31 engages with the internal thread portion 13 formed in the lower end of the buoyancy body 11. Air may remain in the threaded coupling portion, thus affecting the buoyancy of the buoyancy adjustment unit 10.

To eliminate air that has been in the upper end of the perforated pipe body 31 and to provide precise buoyancy in response to variations in the freshwater-saltwater interface, an air discharge hole 34 is formed in the external threaded portion 33, and an air discharge hole 34 is also formed in the internal threaded portion 13 at a corresponding position.

When the external threaded portion 33 completely engages with the internal threaded portion 13, the air discharge hole 34 of the external threaded portion 33 communicates with the air discharge hole 34 of the internal threaded portion 13 so that air that has been in the upper end of the perforated pipe body 31 can be completely discharged to the outside through the air discharge hole 34.

### Mode for Invention

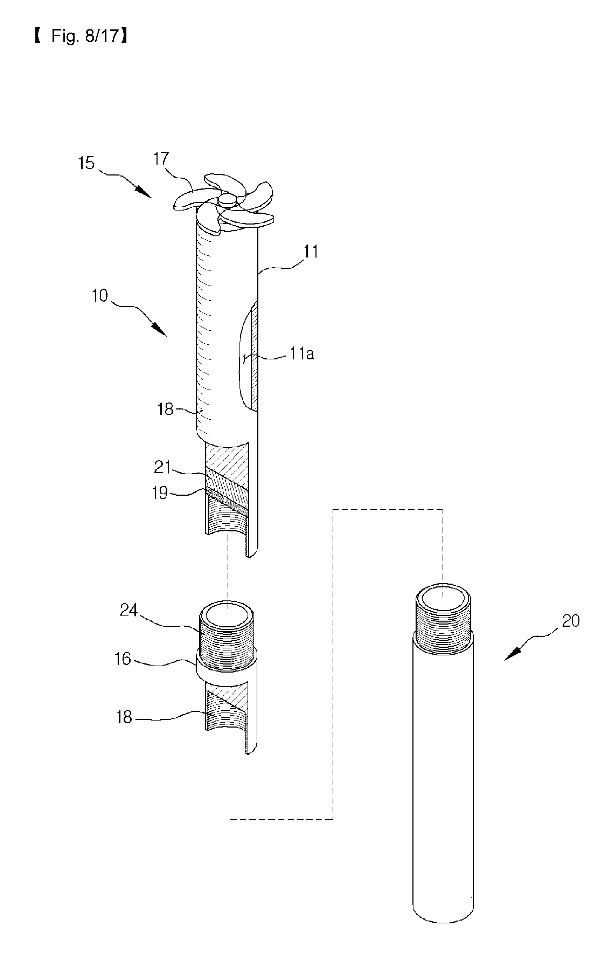

Fig. 8 illustrates another embodiment of the freshwater-saltwater interface position tracking device 1 according to the present invention.

Although the perforated pipe 30 may be removably provided around the circumferential outer surface of the cordless measurement sensor 20 to protect the cordless measurement sensor 20, the present invention may be embodied without using the perforated pipe 30 in such a way that the cordless measurement sensor 20 is directly coupled to the buoyancy adjustment unit 10.

Typically, an external thread may be formed on the circumferential outer surface of an upper end of the cordless measurement sensor 20 so that it can be threadedly coupled to a connector 21.

Alternatively, the measurement sensor 20 which has an external thread on the circumferential outer surface of the upper end thereof may be directly coupled, without using the connector 21, to an internal thread formed in a lower end of the buoyancy adjustment unit 10.

Fig. 8 illustrates this embodiment. The cordless measurement sensor 20 can have a variety of shapes and also be many different sizes. The diameter of the circumferential outer surface of the upper end of the cordless measurement sensor 20 varies depending on the size thereof. A connection member 16 is used to threadedly couple different kinds of cordless measurement sensors 20 to the buoyancy adjustment unit 10 that has a fixed size.

A first thread 24 is formed on a circumferential outer surface of an upper end of the connection member 16, and a second thread 18 is formed on a circumferential inner surface of a lower end of the connection member 16. The second thread 18 engages with the external thread that is formed on the upper end of the cordless measurement sensor 20. The first thread 24 engages with the internal thread that is formed on a circumferential inner surface of the lower end of the buoyancy body 11 of the buoyancy adjustment unit 10. Thereby, the cordless measurement sensor 20 is coupled to the buoyancy adjustment unit 10.

The diameter of the second thread 18 of the connection member 16 may vary depending on the diameter of the cordless measurement sensor 20. The diameter of the first thread 24 is fixed to a value corresponding to that of the circumferential inner surface of the lower end of the buoyancy body 11.

As shown in Fig. 8, the buoyancy adjustment unit 10 comprises the buoyancy body 11 which has an internal space 11a which can contain fluid therein, wherein the buoyancy body 11 has the shape of a pipe (a tube) that is closed on a lower end thereof and has an opening 11b in an upper end thereof so that fluid can be supplied into the buoyancy body 11 through the opening 11b. The internal thread by which the cordless measurement sensor 20 is coupled to the buoyancy body 11 is formed in the circumferential inner surface of the lower end of the buoyancy body 11. A sealing member 19 is provided below the internal space 11a to prevent a thread formed in the lower end of the closed pipe from causing fluid to leak from the interior of the pipe. That is, the sealing member 19 watertightly isolates the internal space 11a from the thread.

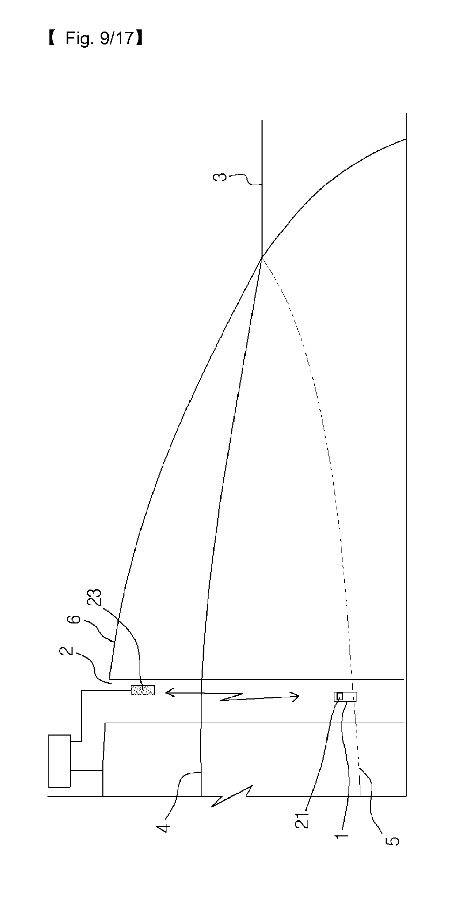

Fig. 9 is a view illustrating a method of measuring variation in the freshwater-saltwater interface using the freshwater-saltwater interface position tracking device according to the present invention.

Typically, an infrared communication device which enables local area communication with a storage medium is embedded in the upper end of the cordless measurement sensor 20. Thus, data that has been stored in the cordless measurement sensor 20 is transmitted to the storage medium.

Therefore, in lieu of using local area communication and transmitting data that has been in the cordless measurement sensor 20 to the storage medium, if a transmitting module 21 is provided with a transmitter that can enable wide area communication with a small DB and is installed above the sealing member 19, and a receiving module 23 is disposed at the beginning of the monitoring well 2, data that is transmitted from the cordless measurement sensor 20 can be collected in real time outside the monitoring well 2. Thereby, variation in the freshwater-saltwater interface 5 can be observed in real time.

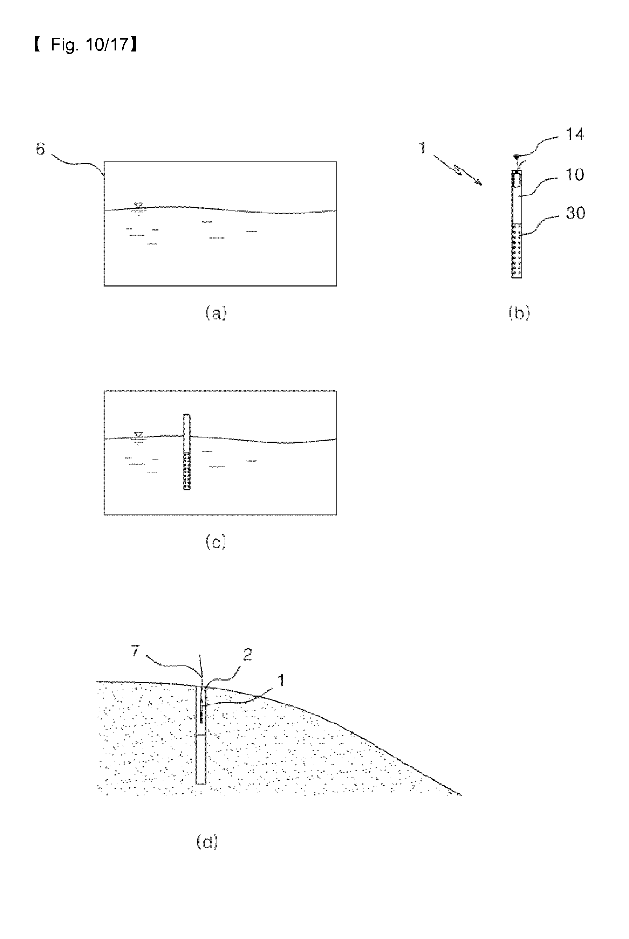

The freshwater-saltwater interface position tracking device 1 is inserted into the formed (bored) monitoring well 2 to measure the variation of freshwater-saltwater interface in the coastal area in the manner shown in Fig. 10. The installation apparatus A for inserting the freshwater-saltwater interface position tracking device 1 into the monitoring well 2 or removing it therefrom will be explained later herein.

After the freshwater-saltwater interface position tracking device 1 that has been assembled is prepared, a water tub 6, which contains water that is adjusted in the salt concentration to create environmental conditions similar to that of a place where the device 1 is installed, is prepared in a place such as a laboratory.

Subsequently, in the prepared water tub 6, fluid 40 is supplied into the internal space 11a of the buoyancy body 11 of the buoyancy adjustment unit 11 so that the buoyancy of the buoyancy adjustment unit 11 is adjusted.

Adjusting the buoyancy of the buoyancy adjustment unit 11 will dispose the freshwater-saltwater interface position tracking device 1 at the freshwater-saltwater interface 5.

It is preferable for the fluid 40 to be pure fluid. Most preferably, distilled water which is pure water is used as the fluid 40.

As such, although the buoyancy of the buoyancy adjustment unit 10 can be adjusted by an experimental method, this method has the disadvantage of having to precisely adjust the salt concentration of water of the water tub 6. Therefore, as shown in Fig. 4, the buoyancy adjustment unit 10 is made of transparent material, and graduations 18 which can indicate the volume of distilled water contained in the internal space of the buoyancy adjustment unit 10 are formed on the circumferential outer surface of the buoyancy adjustment unit 10, whereby the buoyancy of the buoyancy adjustment unit 10 may be predicted by a calculative method.

That is, if the volume of distilled water that is contained in the buoyancy adjustment unit 10 is found, the neutral buoyancy at which the freshwater-saltwater interface position tracking device 1 is disposed at the freshwater-saltwater interface can be calculated by measuring the weight of the freshwater-saltwater interface position tracking device 1. As such, neutral buoyancy can be obtained by simple calculation.

As shown in Fig. 10(d), the freshwater-saltwater interface position tracking device 1 that has had its buoyancy adjusted is inserted into the monitoring well 2 by the installation apparatus A that has the electromagnet on the distal end thereof.

In detail, the freshwater-saltwater interface position tracking device 1 is attached to the electromagnet 100 of the installation apparatus A, is inserted into the monitoring well 2, and then is detached from the electromagnet 100. Then, the device 1 can be stably installed on the freshwater-saltwater interface 5.

As shown in Fig. 2, the freshwater-saltwater interface position tracking device 1 that has been installed in the monitoring well 2 formed in the coastal area moves upwards or downwards depending on variation in the thickness of the underground freshwater and variation in the thickness of the underground saltwater due to tidal phenomenon, thus measuring variation of the freshwater-saltwater interface.

In detail, as shown in Fig. 2, the level 4 of the underground water of the coastal area varies depending on factors such as the amount of pumped water or the amount of recharge of rainfall or variations in the sea level 3 because of the tidal phenomenon. The freshwater-saltwater interface 5 also varies.

Depending on the variation in the level 4 of the underground water and the freshwater-saltwater interface 5, the freshwater-saltwater interface position tracking device 1 is moved by the buoyancy adjustment unit 10 upwards or downwards in the monitoring well 2. The cordless measurement sensor 20 measures variation of a physical, chemical, and biological properties of the water such as water pressure, temperature, electrical conductivity, concentration of chloride, and etc. at the interface and transmits cordless communication signals to the recording device that is installed on the ground so that the observer who is on the ground can monitor variations in the position of the freshwater-saltwater interface.

As stated above, in the freshwater-saltwater interface position tracking device 1, the installation apparatus A can detachably catch, using the electromagnet 100, the sealing cap 14 provided with the variable metal member 15, thus facilitating the installation and removal of the device 1.

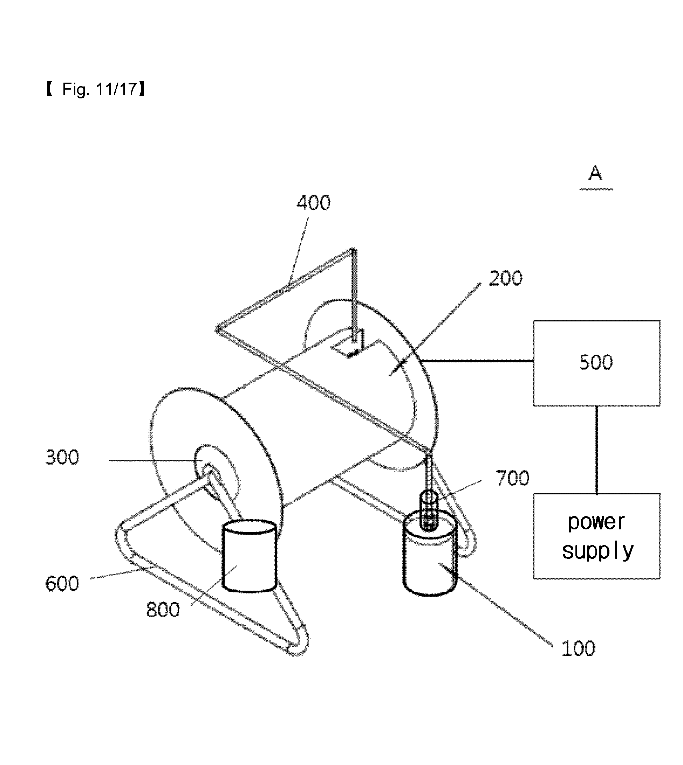

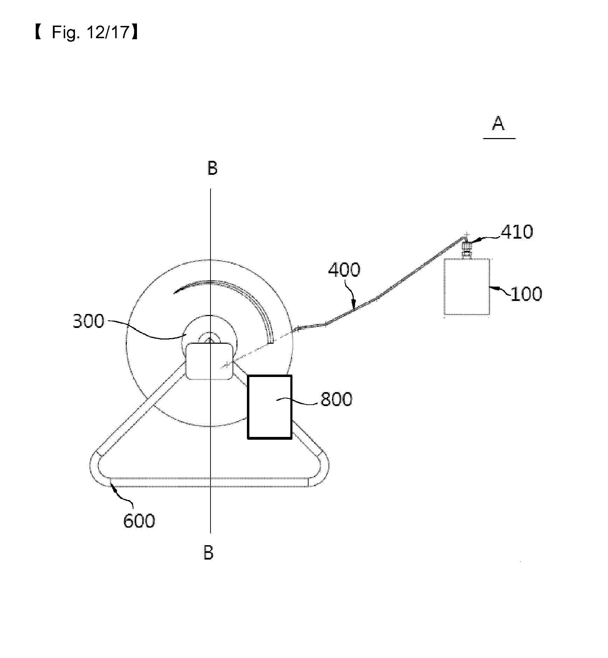

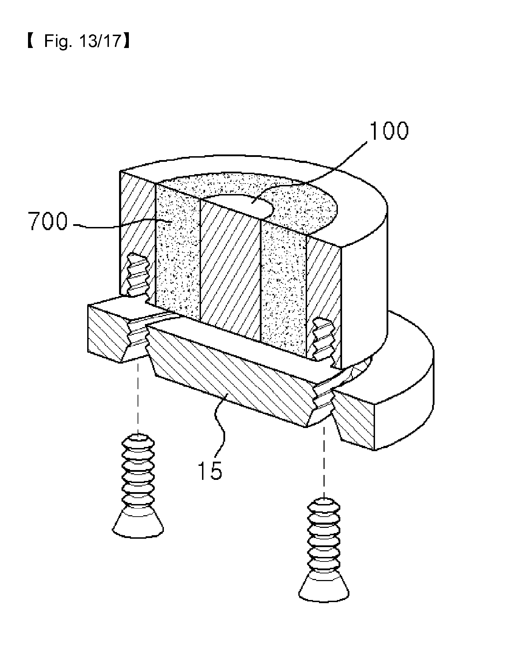

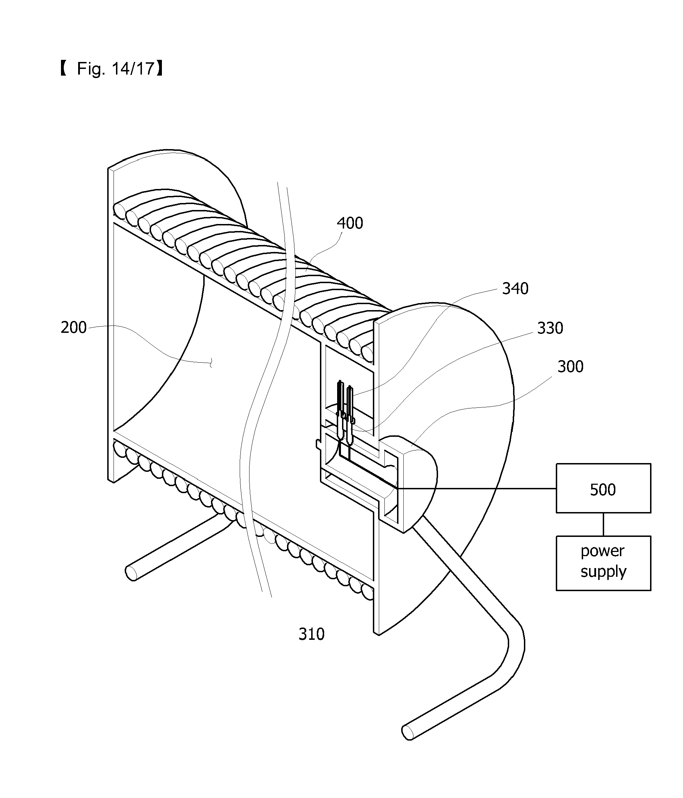

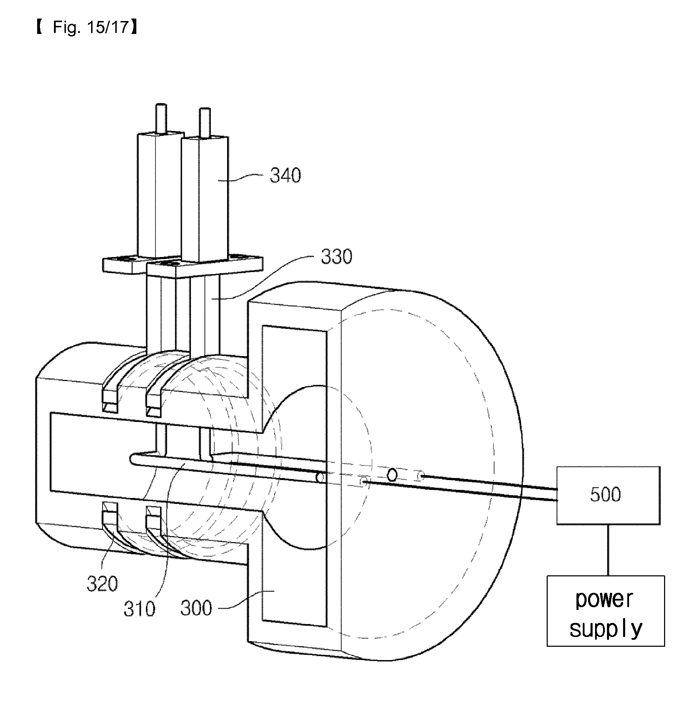

Fig. 11 is a perspective view of the apparatus A for installing the freshwater-saltwater interface position tracking device according to the present invention. Fig. 12 is a side view of the installation apparatus A according to the present invention. Fig. 13 is an enlarged view of an electromagnet of the installation apparatus A according to the present invention. Fig. 14 is a sectional view taken along line B-B to illustrate the installation apparatus A according to the present invention. Fig. 15 is an enlarged view of portion C that illustrates the installation apparatus A according to the present invention.

As shown in Figs. 11 through 15, the apparatus A for installing the freshwater-saltwater interface position tracking device according to the present invention includes an electromagnet 100, a power cable 400, a rotary cylinder 200, a fixed cylinder 300, connection rings 320, first connection bars 330 and a variable metal member 15 which is removably attached to the electromagnet 100.

A first end of the power cable 400 is connected to the electromagnet 100, and the power cable 400 is wound around a circumferential outer surface of the rotary cylinder 200 so that depending on the direction in which the rotary cylinder 200 rotates, the power cable 400 can be wound or unwound.

The fixed cylinder 300 is disposed in the center of the rotary cylinder 200 and functions as a rotary shaft of the rotary cylinder 200. The fixed cylinder 300 has a hollow shape. The connection rings 320 are fitted over a circumferential outer surface of the fixed cylinder 300. Each connection ring 320 is fixed such that it passes through from the circumferential inner surface to the circumferential inner surface of the fixed cylinder 300.

A second end of the power cable 400 is connected to first ends of the first connection bars 330. The first connection bars 330 are fixed in the rotary cylinder 200 so that the first connection bars 330 rotate along with the rotary cylinder 200. Second ends of the first connection bars 330 are connected to the respective connection rings 320 that are fixed in the fixed cylinder 300 such that the contact between the first connection bars 300 and the connection rings 320 are continuously maintained without disconnection.

Even when the first connection bars 330 rotate, they must maintain the connection to the connection rings 320. For this, each first connection bar 330 is coupled to a spring (not shown) so that the first connection bar 330 can be biased to the connection ring 320 by elastic force of the spring.

Each first connection bar 330 is a conductor, and an insulator 340 covers a portion of the first connection bar 330, other than portions which make contact with the corresponding connection rings 320 and are connected to the power cable 400. The spring is disposed in the insulator 340.

The first connection bar 330 is covered with the insulator 340 so that electric current can be prevented from flowing through elements other than the electromagnet when power is applied thereto.

The connection rings 320 are connected to respective second connection bars 310 on a circumferential inner surface of the hollow fixed cylinder 300. The second connection bars 310 are electrically connected to a power supply through a side surface of the fixed cylinder 300 so that power can be applied from the power supply to the second connection bars 310.

The second connection bars 310 are fixed in the hollow space of the fixed cylinder 300. Here, the term "electrically connected" means that power supplied from the power supply is applied to the second connection bars 310 which are conductors so that current can flow through the second connection bars 310.

The second connection bars 310 and the connection rings 320 may be an integrated metal member, and they are fixed to the fixed cylinder 300. Power applied from the power supply is supplied to the connection rings 320 so that the power is applied to the electromagnet 100 via the power cable 400 by the first connection bars 330 which rotate around the connection rings 320 and maintain contact with the connection rings 320, whereby the electromagnet 100 forms a magnetic field.

A control unit 500 is installed between the second connection bars 310 and the power supply. The control unit 500 controls resistance and varies the magnitude of the magnetic field, thus controlling the magnitude of the electromagnet 100.

A support 600 is coupled to opposite side surfaces of the fixed cylinder 300 so that the installation apparatus A can be supported on the ground by the support 600.

Furthermore, a storage receptacle 800 is mounted to the support 600 so that the electromagnet 100 can be stored in the storage receptacle 800, thus enhancing the portability of the installation apparatus A.

The electromagnet 100 must come into contact with fresh water and salt water when installing the freshwater-saltwater interface position tracking device 1 in the monitoring well 2 or removing it therefrom. Moreover, the electromagnet 100 is immersed in water to a depth corresponding to the freshwater-saltwater interface. That is, it must withstand the water pressure at a depth of the freshwater-saltwater interface in a coastal area where it is supposed to be installed. Therefore, a watertight sealing member 700 covers and seals the electromagnet 100 to prevent it from corroding and protect it from high water pressure.

Furthermore, not only the electromagnet 100 but also a connector 410 which connects the power cable 400 to the electromagnet 100 may be covered with the watertight sealing member 700 so that they can stay watertight.

The structure in which the watertight sealing member 700 covers both the electromagnet 100 and the connector 410 can enhance the corrosion prevention function and the pressure-resistance characteristics and prevent a short of the power cable 400 at the connector 410 which may cause the current to flow through water, resulting in an electric shock accident.

As shown in Fig. 13, the variable metal member 15 is removably attached to the outer surface of the watertight sealing member 700.

The structure of the variable metal member 15 may be the same as that of Fig. 5 in which metal blades 17 can be folded and unfolded. Alternatively, as shown in Fig. 13, the variable metal member 15 may be embodied by an additional metal plate.

The variable metal member 15 may be disposed outside the watertight sealing member 700 that covers the electromagnet 100 or directly connected to a lower end of the electromagnet 100.

The magnetic force of the electromagnet 100 is transmitted to the variable metal member 15 so that the entirety of the variable metal member 15 can function as an electromagnet. As described above with reference to Fig. 5, the variable metal member 15 is used to make the diameter of the electromagnet similar to that of the monitoring well 2 so that finding the freshwater-saltwater interface position tracking device 1 in the monitoring well 2 can be facilitated regardless of the diameter of the monitoring well 2.

It is easy to wind the power cable 400 of the installation apparatus A around the rotary cylinder 200 or unwind it therefrom. Therefore, the installation apparatus A is moved after the power cable 400 has been wound. When it is required to install the freshwater-saltwater interface position tracking device 1 in the monitoring well 2, the device 1 is attached to the electromagnet of the installation apparatus A and then the power cable 400 is unwound from the rotary cylinder 200 so that the device 1 moves downwards in the monitoring well 2.

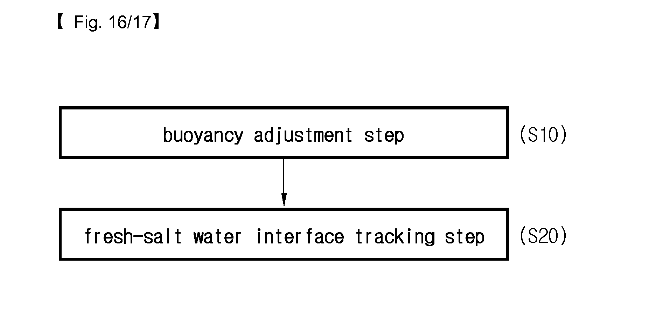

Fig. 16 is a flowchart of a method of tracking the position of a freshwater-saltwater interface according to the present invention.

As shown in Fig. 16, the method of tracking the position of the freshwater-saltwater interface of the underground water using the freshwater-saltwater interface position tracking device 1 includes a buoyancy adjustment step S10 of supplying distilled water into the buoyancy adjustment unit 10 of the freshwater-saltwater interface position tracking device 1 and adjusting the buoyancy of the device 1 so that the device 1 is disposed at the freshwater-saltwater interface, and a freshwater-saltwater interface tracking step S20 of tracking variation of the position of the freshwater-saltwater interface using the cordless measurement sensor 20. As described above, the freshwater-saltwater interface position tracking device 1 includes the buoyancy adjustment unit 10 which has the shape of a pipe that has an internal space therein and is closed on the lower end thereof; the sealing cap 14 which is removably coupled to the upper end of the buoyancy adjustment unit 10; the variable metal member 15 which is attached to the sealing cap 14; the cordless measurement sensor 20 which is installed under the lower end of the buoyancy adjustment unit 10; and the perforated pipe 30 which is coupled to the lower end of the buoyancy adjustment unit 10 and covers the cordless measurement sensor 20.

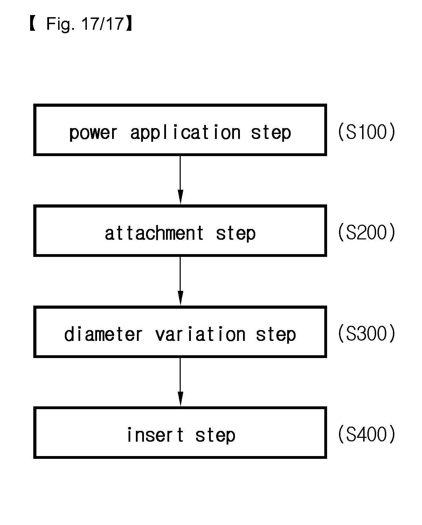

Fig. 17 is a flowchart of a method of installing the freshwater-saltwater interface position tracking device according to the present invention.

As shown in Fig. 17, the method of installing the freshwater-saltwater interface position tracking device using the installation apparatus A includes a power application step S100 of applying power to the connection rings 320 of the installation apparatus A so that a magnetic field is formed on the electromagnet 100 by the applied power, thus magnetizing the electromagnet 100, an attachment step S200 of attaching the freshwater-saltwater interface position tracking device 1 to the magnetized electromagnet 100, a diameter variation step S300 of varying the diameter of the variable metal member 15 depending on the diameter of the monitoring well 2, and an insert step S400 of inserting the freshwater-saltwater interface position tracking device 1 into the monitoring well 2. As stated above, the installation apparatus A includes the power cable 400; the rotary cylinder 200 which is rotatably provided and around which the power cable 400 is wound; the fixed cylinder 300 which is disposed in the central portion of the rotary cylinder 200 and functions as the rotary shaft of the rotary cylinder 200; the electromagnet 100 which is connected to the first end of the power cable 400; the variable metal member 15 which is attached to the lower end of the electromagnet 100; the first connection bars 330 which are connected to the second end of the power cable 400 and are fixed in the rotary cylinder 200; and the connection rings 320 which are fixed in the fixed cylinder 300 such that the circumferential outer surfaces thereof make contact with the respective first connection bars 330.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

### Industrial Applicability

As described above, in a device for tracking the position of a freshwater-saltwater interface of underground water in a coastal area using a neutral buoyancy mechanism according to the present invention, a buoyancy adjustment unit which uses fluid and adjusts the buoyancy is provided with a cordless measurement sensor so that the device moves in response to variations in the position of a freshwater-saltwater interface. Thus, the device can take more precise real-time measurements of the level, temperature, conductivity, etc. of the underground water of the coastal area. Therefore, the present invention can be very useful in this industry.

## Claims

1. A device for tracking a position of a freshwater-saltwater interface of underground water in a coastal area in such a way that a cordless measurement sensor is inserted, to track a position of a freshwater-saltwater interface, into a underground water monitoring well that is formed to measure the underground water, the device comprising:
a buoyancy adjustment unit comprising a pipe having an internal space, the buoyancy adjustment unit being closed on a lower end thereof;
a sealing cap removably coupled to an upper end of the buoyancy adjustment unit;
a variable metal member attached to the sealing cap;
a cordless measurement sensor provided under the lower end of the buoyancy adjustment unit; and
a perforated pipe coupled to the lower end of the buoyancy adjustment unit, the perforated pipe covering the cordless measurement sensor,
wherein distilled water is supplied into the buoyancy adjustment unit, and a buoyancy of the buoyancy adjustment unit is adjusted depending on an amount of distilled water.

2. The device for tracking the position of the freshwater-saltwater interface of the underground water according to claim 1, wherein the variable metal member is extendable in a circumferential direction depending on a diameter of the underground water monitoring well.

3. The device for tracking the position of the freshwater-saltwater interface of the underground water according to claim 1, wherein the buoyancy adjustment unit is made of a transparent material, and graduations are marked on an outer surface of the buoyancy adjustment unit, whereby the buoyancy of the buoyancy adjustment unit is adjusted by calculating the amount of distilled water supplied into the buoyancy adjustment unit depending on a weight of the device.

4. The device for tracking the position of the freshwater-saltwater interface of the underground water according to claim 3, wherein an air discharge hole is formed in a connector connecting the perforated pipe to the buoyancy adjustment unit so that air is able to be discharged out of the perforated pipe through the air discharge hole.

5. A device for tracking a position of a freshwater-saltwater interface of underground water in a coastal area in such a way that a cordless measurement sensor is inserted, to track a position of a freshwater-saltwater interface, into a underground water monitoring well that is formed to measure the underground water, the device comprising:
a buoyancy adjustment unit comprising a pipe having an internal space, the buoyancy adjustment unit being closed on a lower end thereof;
a sealing cap removably coupled to an upper end of the buoyancy adjustment unit;
a cordless measurement sensor provided under the lower end of the buoyancy adjustment unit; and
a connection member provided under the lower end of the buoyancy adjustment unit, the connection member being connected to the cordless measurement sensor,
wherein distilled water is supplied into the buoyancy adjustment unit, and a buoyancy of the buoyancy adjustment unit is adjusted depending on an amount of distilled water.

6. An apparatus for installing or removing a device for tracking a position of a freshwater-saltwater interface of underground water, the apparatus comprising:
a power cable;
a rotary cylinder around which the power cable is wound, the rotary cylinder being provided so as to be rotatable;
a fixed cylinder disposed at a center of the rotary cylinder, the fixed cylinder functioning as a rotary shaft of the rotary cylinder;
an electromagnet connected to a first end of the power cable;
a first connection bar connected to a second end of the power cable, the first connection bar fixed in the rotary cylinder;
a connection ring having a circumferential outer surface in contact with the first connection bar, the connection ring provided in the fixed cylinder;
a watertight sealing member covering the electromagnet; and
a variable metal member that is removably attached to the electromagnet.

7. The device for tracking the position of the freshwater-saltwater interface of the underground water according to claim 6, wherein the connection ring is connected to a second connection bar on a circumferential inner surface of the fixed cylinder,
wherein the second connection bar is electrically connected to a power supply connected to the fixed cylinder so that power is applied from the power supply to the second connection bar.

8. The device for tracking the position of the freshwater-saltwater interface of the underground water according to claim 7, wherein the second connection bar is disposed in a hollow space of the fixed cylinder.

9. The device for tracking the position of the freshwater-saltwater interface of the underground water according to claim 8, wherein the first connection bar, the connection ring and the second connection bar comprise conductors.

10. The device for tracking the position of the freshwater-saltwater interface of the underground water according to claim 6, wherein
the first connection bar comprises a covering part that is an insulator,
a spring is provided in the covering part so that the contact between the connection ring and the first connection bar that is rotating around the connection ring is maintained by elastic force of the spring.

11. A method of tracking a position of a freshwater-saltwater interface of underground water using a freshwater-saltwater interface position tracking device comprising: a buoyancy adjustment unit comprising a pipe having an internal space, the buoyancy adjustment unit being closed on a lower end thereof; a sealing cap removably coupled to an upper end of the buoyancy adjustment unit; a variable metal member attached to the sealing cap; a cordless measurement sensor provided under the lower end of the buoyancy adjustment unit; and a perforated pipe coupled to the lower end of the buoyancy adjustment unit, the perforated pipe covering the cordless measurement sensor, the method comprising:
supplying distilled water into the buoyancy adjustment unit and adjusting a buoyancy of the buoyancy adjustment unit so that the device is disposed at a freshwater-saltwater interface of the underground water; and
tracking variation in a position of the freshwater-saltwater interface using the cordless measurement sensor.

12. A method of installing a freshwater-saltwater interface position tracking device using an installation apparatus, the installation apparatus comprising: a power cable; a rotary cylinder around which the power cable is wound, the rotary cylinder being provided so as to be rotatable; a fixed cylinder disposed at a center of the rotary cylinder, the fixed cylinder functioning as a rotary shaft of the rotary cylinder; an electromagnet connected to a first end of the power cable; a variable metal member attached to a lower end of the electromagnet; a first connection bar connected to a second end of the power cable, the first connection bar fixed in the rotary cylinder; and a connection ring having a circumferential outer surface in contact with the first connection bar, the connection ring provided in the fixed cylinder, the method comprising:
applying power to the connection ring of the installation apparatus so that a magnetic field is formed on the electromagnet by the applied power, thus magnetizing the electromagnet;
attaching the freshwater-saltwater interface position tracking device to the magnetized electromagnet;
varying a diameter of the variable metal member depending on a diameter of the underground water monitoring well; and
inserting the freshwater-saltwater interface position tracking device into the underground water monitoring well.
